# EUROPEAN PATENT SPECIFICATION

(11) **EP 2 419 097 B1**
(45) Date of publication and mention of the grant of the patent: **25.09.2013**
(21) Application number: 10764570.7
(22) Date of filing: 16.04.2010
(51) Int. Cl.: A61K 31/382, A61K 31/155, A61K 31/4439, A61K 31/64, A61K 45/00, A61P 3/10, A61P 5/50, A61P 9/10, A61P 9/14, A61P 13/12, A61P 25/00, A61P 27/02, A61P 43/00, C07D 335/02

(54) **PHARMACEUTICAL COMPOSITION COMPRISING (1S)-1,5-ANHYDRO-1-[5-(4-ETHOXYBENZYL)-2-METHOXY-4-METHYLPHENYL]-1-THIO-D-GLUCITOL AND METFORMIN AND USES THEREOF IN THE TREATMENT OF DIABETES**
PHARMAZEUTISCHE ZUSAMMENSETZUNG ENTHALTEND (1S)-1,5-ANHYDRO-1-[5-(4-ETHOXYBENZYL)-2-METHOXY-4-METHYLPHENYL]-1-THIO-D-GLUCITOL UND METFORMIN UND DEREN VERWENDUNG ZUR BEHANDLUNG VON DIABETES
COMPOSITION PHARMACEUTIQUE COMPRENANT(1S)-1,5-ANHYDRO-1-[5-(4-ETHOXYBENZYL)-2-METHOXY-4-METHYLPHENYL]-1-THIO-D-GLUCITOL ET METFORMIN ET SON UTILISATION POUR TRAITER LE DIABÈTES

(30) Priority: 16.04.2009 JP 2009100210
(43) Date of publication of application: 22.02.2012
(60) Divisional application: 13157571.4
(73) Proprietor: Taisho Pharmaceutical Co., Ltd., Tokyo 170-8633 (JP)
(72) Inventor: TAKAHASHI, Teisuke, Tokyo 170-8633 (JP); UCHIDA, Saeko, Tokyo 170-8633 (JP)
(74) Representative: HOFFMANN EITLE
(86) International application number: PCT/JP2010/057196
(87) International publication number: WO 2010/119990

(56) References cited:
- EP-A1- 1 845 095
- EP-A1- 1 845 095
- EP-A1- 2 036 901
- WO-A1-2008/020011
- WO-A1-2008/072726
- JP-A- 2008 031 161
- US-A1- 2003 064 935

## Description

### Technical Field

The present invention relates to pharmaceutical compositions useful in the treatment of diabetes mellitus, diseases associated with diabetes mellitus, or complications of diabetes mellitus. More particularly, the present Invention relates to pharmaceutical compositions comprising a combination of (A) (1S)-1,5-anhydro-1-[5-(4-ethoxybenzyl)-2-methoxy-4-methylphenyl]-1-thio-D-glucitol or a pharmaceutically acceptable salt thereof or a hydrate of the compound or the salt; and (B) metformin.

### Background Art

Diabetes mellitus is a group of diseases mainly characterized by chronic hyperglycemia due to insufficiency of insulin action and is accompanied by various metabolic abnormalities. The primary treatment of diabetes mellitus consists of exercise and diet therapy; if these methods are incapable of adequate glycemic control, drug therapy with oral antidiabetic agents is conducted. However, depending on the condition of a diabetic patient, treatment with a single drug sometimes encounters difficulty in achieving good glycemic control, and what is more, the development of side effects prevents the drug from being used in an adequate dose or over an adequate period of time. According to one literature report, a biguanide solely used on patients with type 2 diabetes gave only about 25% probability for the possibility of lowering the value of glycated hemoglobin (HbAlc) to 7% or less (see NPL 1). It is also known that if a sulfonylurea is used alone, side effects such as the development of hypoglycemia and weight gain are not the sole problems but that its action of improving hyperglycemia becomes inadequate in patients whose ability to secrete insulin have been deteriorated by failure of beta-cells with the progress of diabetic condition. It is also known that if an insulin sensitizer is used alone, there occurs the problem of occasional development of such side effects as weight gain, edema, heart failure, and hepatopathy. It is also known that if insulin is used alone, there occur the problems of hypoglycemia and weight gain. It is also known that if an α-glucosidase inhibitor is used alone, there occurs the problem of occasional development of abdominal symptoms as side effects. In addition, when a GLP-1 mimetic is used alone, nausea and vomiting are known as therapeutic problems. Furthermore, it is known that patients with type 2 diabetes, when they are exposed to a hyperglycemic condition for a prolonged period of time, will suffer a deterioration in the function of beta-cells; however, most antidiabetic agents are incapable of adequately suppressing the deterioration in the function of beta-cells even if the blood glucose levels are lowered.
As a means of solving the aforementioned problems involved in the treatment with single drugs, a combination therapy is under review that uses a combination of antidiabetic agents that act by different mechanisms. However, few combinations are capable of diminishing the aforementioned problems with the use of single drugs. For example, it is known that the risk for the development of hypoglycemia, a recognized side effect of sulfonylureas, is further increased if they are used in combination with other antidiabetic agents. In addition, the weight gain recognized from sulfonylureas and insulin sensitizers cannot usually be controlled by using them in combination with other antidiabetic agents. Hence, a novel combination of plural pharmaceutical drugs is in demand that can achieve a good glycemic control and which yet causes no appreciable side effects.

Glucose in the blood is filtered in the glomeruli of each kidney and then reabsorbed as mediated by sodium-dependent glucose cotransporters (SGLTs) located at the beginnings of proximal tubules. SGLT2 inhibitors typified by a 1-thio-D-glucitol compound (see PLT 1) inhibit the activity of SGLT2 having low glucose affinity but high glucose transport capacity, so they exhibit a blood glucose reducing action in various animal models by promoting the excretion of urinary glucose.

It was recently reported that side effects of insulin sensitizers could be reduced by combining them with SGLT2 inhibitors (see PLT 2) but there has been no report of a pharmaceutical composition that combines an SGLT2 inhibitor with an insulin sensitizer in order to provide an enhanced blood glucose lowering action. There is also literature teaching the combined use of an SGLT2 inhibitor and a dipeptidyl peptidase IV inhibitor (see PLT 3 and 4). However, no report has been made of pharmaceutical compositions of the type contemplated by the present invention that comprise combinations of SGLT2 inhibitors with at least one member of the group consisting of biguanides, insulin secretagogues, insulin sensitizers, insulins, dipetidyl peptidase IV inhibitors, α-glucosidase inhibitors, and GLP-1 mimetics.

### Citation List

### Patent Literature

PLT 1: Official Gazette of International Publication WO 2006/073197 A1
PLT 2: Patent Official Gazette of EP 1381361 B1
PLT 3: Official Gazette of International Publication WO 2009/022007 Al
PLT 4: Official Gazette of International Publication WO 2009/022010 Al (corresponding to EP 1 845 095 Al)

### Non Patent Literature

NPL 1: Tonyobyougaku (Kiso to Rinshou) [Diabetology (Fundamentals and Clinical Applications)], pp.949-954, 2007, Nishimura Shoten

### Summary of Invention

An object of the present invention is to provide a novel pharmaceutical composition comprising a combination of plural drugs that has superior efficacy in preventing or treating diabetes mellitus, diseases associated with diabetes mellitus, or complications of diabetes mellitus and which yet causes no appreciable side effects.
Another object of the present invention is to provide a Pharmaceutical compositon for use in preventing or treating diabetes mellitus, diseases associated with diabetes mellitus, or complications of diabetes mellitus using the pharmaceutical composition.

The present inventors found that when a specific 1-thio-D-glucitol compound having an SGLT2 inhibiting action or a pharmaceutically acceptable salt thereof or a hydrate of the compound or the salt was administered in combination with metformin, a marked blood glucose lowering action, suppression of the failure of pancreatic β-cells, and alleviation of side effects were caused; the present invention has been accomplished on the basis of this finding.

Thus, the present invention provides the following:
(1) A pharmaceutical composition comprising a combination of
   (A) (1S)-1,5-anhydro-1-[5-(4-ethoxybenzyl)-2-methoxy-4-methylphenyl]-1-thio-D-glucitol (hereinafter also referred to as "compound A" or as "1-thio-D-glucitol compound A") or a pharmaceutically acceptable salt thereof or a hydrate of the compound or the salt; and
   (B) metformin.
(2) The pharmaceutical composition according to (1), wherein the metformin is present in the form of metformin hydrochloride.
(3) A combination of:
   (A) (1S)-1,5-anhydro-1-[5-(4-ethoxybenzyl)-2-methoxy-4-methylphenyl]-1-thio-D-glucitol or a pharmaceutically acceptable salt thereof or a hydrate of the compound or the salt; and
   (B) metformin
   for use in a method of preventing or treating diabetes mellitus, diseases associated with diabetes mellitus, or complications of diabetes mellitus, wherein (A) and (B) are administered to a patient in need either simultaneously or separately.
(4) The combination for use according to (3), wherein the metformin is present in the form of metformin hydrochloride.
(5) The combination for use according to (3) or (4), wherein diabetes mellitus is type 2 diabetes.
(6) The combination for use according to any one of (3) to (5), wherein the complication of diabetes mellitus is diabetic retinopathy, diabetic nephropathy, diabetic neuropathy, cerebrovascular disorder, ischemic cardiac disease, or peripheral arterial disease.

### Advantageous Effects of Invention

The pharmaceutical composition of the present invention comprising plural pharmaceutical drugs showed superior glycated hemoglobin (GHb) value lowering action and plasma glucose level lowering action over the individual drugs. In addition, the pharmaceutical composition of the present invention comprising plural pharmaceutical drugs suppressed the lowering of plasma insulin levels that might have occurred with the progress of diabetic condition. Furthermore, the pharmaceutical composition of the present invention comprising plural pharmaceutical drugs alleviated the side effects of the individual drugs (i.e., weight gain and hypoglycemia).

### Description of Embodiments

The "pharmaceutically acceptable salt" may be exemplified by: salts with inorganic acids such as sulfuric acid, hydrochloric acid, hydrobromic acid, phosphoric acid, and nitric acid; salts with organic acids such as acetic acid, oxalic acid, lactic acid, tartaric acid, fumaric acid, maleic acid, citric acid, benzenesulofnic acid, methanesulfonic acid, p-toluenesulfonic acid, benzoic acid, camphorsulfonic acid, ethanesulfonic acid, glucoheptonic acid, gluconic acid, glutamic acid, glycolic acid, malic acid, malonic acid, mandelic acid, galactaric acid, and naphthalene-2-sulfonic acid; salts with one or more metal ions such as lithium ion, sodium ion, potassium ion, calcium ion, magnesium ion, zinc ion, and aluminum ion; and salts with amines such as ammonia, arginine, lysine, piperazine, choline, diethylamine, 4-phenylcyclohexylamine, 2-aminoethanol, and benzathine.

The compound A of the present invention can exist as various solvates. They may also be hydrates in view of their applicability as pharmaceutical drugs.
The methods of producing the 1-thio-D-glucitol compounds of formula (I) which are used in the present invention, as well as the pharmaceutically acceptable salts thereof and the hydrates of the compounds or salts which are also used in the present invention are disclosed in the official gazette of International Publication WO 2006/073197 Al.
The 1-thio-D-glucitol compound is used because it exhibits a superior SGLT2 inhibiting activity:
(1S)-1,5-anhydro-1-[5-(4-ethoxybenzyl)-2-methoxy-4-methylphenyl]-1-thio-D-glucitol (formula (A)). 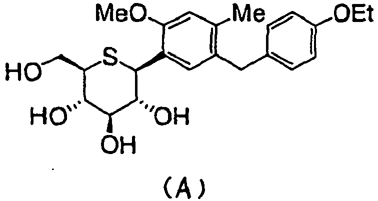
The (1S)-1,5-anhydro-1-[5-(4-ethoxybenzyl)-2-methoxy-4-methylphenyl]-1-thio-D-glucitol, is preferably a hydrate.

Therefore the present invention is a pharmaceutical composition comprising the 1-thio-D-glucitol compound A or a pharmaceutically acceptable salt thereof or a hydrate of the compound or salt and the biguanide metformin. This pharmaceutical composition is a medicine against diabetes mellitus, diseases associated with diabetes mellitus, or complications of diabetes mellitus.

Biguanides are drugs having actions of suppression of hepatic gluconeogenesis, increase of insulin sensitivity in the peripheral tissues, inhibition of glucose absorption from the intestine. Metformin is used the viewpoints of blood glucose lowering action, limited side effects, and the like, with metformin hydrochloride being particularly preferred. These biguanides are known substances and metformin and metformin hydrochloride in particular are disclosed in Emil A. Werner and James Bell, J. Chem. Soc., 121, 1922, 1790-1794, and a commercial product having the trademark GLUCOPHAGE may be used.

"Diabetes mellitus" encompasses type 1 diabetes, type 2 diabetes, and other types of diabetes mellitus due to specific causes. The diseases targeted by the pharmaceutical drugs of the present invention are preferably type 1 diabetes and type 2 diabetes.

"Diseases associated with diabetes mellitus" include obesity, hyperinsulinemia, dysglycemia (glucose metabolism disorder), hyperlipidemia, hypercholesterolemia, hypertriglyceridemia, dyslipidemia (lipid metabolism disorder), hypertension, congestive heart failure, edema, hyperuricemia, and gout.
"Complications of diabetes mellitus" are classified into acute and chronic ones.
"Acute complications" include hyperglycemia (e.g., ketoacidosis), hyperglycemic hyperosmolar syndrome, lactic acidosis, hypoglycemia, and infectious diseases (e.g., skin, soft tissue, biliary, respiratory, and urinary-tract infections).
"Chronic complications" include microangiopathy (diabetic retinopathy, diabetic neuropathy, and diabetic nephropathy), as well as macroangiopathy (cerebrovascular disorder, ischemic heart disease, and peripheral arterial disease).

"Treatment" means administering the pharmaceutical compositions of the present invention to patients who have already manifested diseases such as diabetes mellitus, diseases associated with diabetes mellitus, or complications of diabetes mellitus. Acts of this treatment cover symptomatic therapy intended to mitigate the symptoms derived from the above-mentioned diseases. Also included are treatments for partial or complete recovery from disease, as well as treatments that stop or retard the progress of disease.

"Prevention" means a practice by which patients who have a risk for manifesting diseases such as diabetes mellitus, diseases associated with diabetes mellitus, or complications of diabetes mellitus are administered the pharmaceutical compositions of the present invention before the disease manifests itself.

The pharmaceutical composition of the present invention can be such that the active ingredients described above are formulated as a single preparation (combination preparation) or they are separately formulated as two or more preparations. These preparations may be tablets, granules, powders, capsules, emulsions, suspensions or syrups or alternatively they may be injections in such forms as a sterile solution or a liquid suspension, all being obtained by commonly employed means. If the active ingredients are separately formulated as two or more preparations, the respective preparations may be administered either simultaneously or at given time intervals spaced apart. The two or more preparations may be administered at different frequencies in a day. The pharmaceutical composition of the present invention may be administered either systemically or topically by an oral or parenteral route. If the active ingredients are separately formulated as two or more preparations, the respective preparations may be administered by different routes.

If the pharmaceutical composition of the present invention is formulated as two different preparations, they are highly likely to be administered either simultaneously or at a very short interval, so a document such as a package insert or a sales brochure that accompanies a commercial product preferably states to the effect that the two preparations should be administered in combination. Another preferred embodiment is a kit of two preparations, one comprising the 1-thio-D-glucitol compound A and the other comprising metformin.

The dosage of the pharmaceutical composition of the present invention varies with the target, method, etc. of administration. Take, for example, the case of oral administration; a patient with diabetes mellitus is preferably administered the following daily doses:
(1) 0.1-50 mg, preferably 0.5-25 mg, of the 1-thio-D-glucitol compound A;
(2) 100-3000 mg, preferably 300-3000 mg, of metformin;
Biguanides are usually administered in two or three divided portions. On the other hand, the 1-thio-D-glucitol compound A can be rendered to exhibit a sustained SGLT2 inhibiting action for an extended period. Hence, to design a combination preparation of a type that is to be administered once a day, the 1-thio-D-glucitol compound A of the present invention is preferably used in combination with "a biguanide (metformin) designed to be capable of sustained release."

The "biguanide (metformin) designed to be capable of sustained release" can be prepared in accordance with known methods. For example, biguanides can be rendered to be capable of sustained release by using the slow-releasing method described in the official gazette of WO 96/08243 A1 or the method described in the official gazette of JP 2003-520759 A.

The preparations described above are preferably oral preparations such as tablets, granules, powders, capsules, emulsions, suspensions, and syrups. Specifically, the active ingredients described above may be mixed, either simultaneously or separately, with excipients such as mannitol and lactose and, after granulation, filled into capsules either directly or after being mixed with other additives for oral administration, which are specifically exemplified by excipients (e.g., sugar-based or sugar alcoholic excipients such as glucose, sucrose, mannitol, lactose, xylitol, sorbitol, maltitol, and pullulan; cellulosic excipients such as microcrystalline cellulose; starch-based excipients such as corn starch; inorganic excipients such as anhydrous dibasic calcium phosphate), binders (e.g., cellulosic binders such as methylcellulose, hydroxypropyl cellulose, and hydroxypropyl methylcellulose), disintegrants (e.g., cellulosic disintegrants such as caxmellose calcium, low-substitution hydroxypropyl cellulose, and croscarmelose sodium, or starch-based disintegrants such as partially gelatinized starch and carboxymethyl starch sodium), fluidizers (e.g., inorganic fluidizers such as light silicic anhydride), or lubricants (e.g., stearic acid, magnesium stearate, calcium stearate, talc, and sodium stearyl fumarate); alternatively, the granulation may be pelletized into tablets.

The proportion at which the 1-thio-D-glucitol compound A of the present invention or a pharmaceutically acceptable salt thereof or a hydrate of the compound or salt is combined with metformin varies with the target, method, etc. of administration. Take, for example, the case of administering the pharmaceutical composition of the present invention to a human; if one part by mass of the 1-thio-D-glucitol compound A is combined with 0.01-1000 parts by mass of metformin, it is possible to obtain a superior blood glucose lowering action than is achieved by administering the individual drugs. It is particularly preferred to combine one part by mass of the 1-thio-D-glucitol compound A in combination with 0.1-100 parts by mass of metformin. This enables satisfactory efficacy to be obtained by using the respective drugs in smaller amounts than when they are administered individually. As a further advantage, the pharmaceutical composition of the present invention has fewer side effects because it does not cause hypoglycemia or weight gain that might result from excessive insulin secretion.

Patients who are to be treated by the pharmaceutical composition of the present invention are those who are unable to have good glycemic control in spite of continued diet and exercise and who therefore need be subjected to drug therapy; preferred patients are those who are unable to have good glycemic control in spite of being administered with a single oral antidiabetic drug and who therefore need to take another drug that acts by a different mechanism.

The pharmaceutical composition of the present invention can typically be produced according to the following formulas.

### (Preparation 1) Tablet

Tablets with a diameter of 13 mm were prepared; each tablet contained:

| | |
|---|---|
| (1S)-1,5-anhydro-1-[5-(4-ethoxybenzyl)-2-methoxy-4-methylphenyl]-1-thio-D-glucitol (hereinafter referred to as compound A) | 5 mg |
| Metformin hydrochloride | 500 mg |
| Microcrystalline cellulose | 70 mg |
| Hydroxypropyl cellulose | 25 mg |
| Carboxymethyl starch sodium | 30 mg |
| Magnesium stearate | 3 mg. |

### Examples

On the following pages, the present invention is described in greater detail by means of examples.

### Test Example 1

### <Test item>

Effects of chronic combination treatment of compound A and metformin hydrochloride in diabetic mice

### <Test method>

Eight male db/db mice/group (11-week old; CLEA JAPAN, Inc.) were orally administered with repeated doses of compound A (3 mg/kg, once daily) and a biguanide, metformin hydrochloride (50 mg/kg or 150 mg/kg, twice daily; SIGMA-ALDRICH JAPAN K.K.), either alone or in combination, for 27 days. Eight db/m mice (11-week old; CLEA JAPAN, Inc.) were used as nondiabetic normal controls.

Before and 27th day after the start of repeated administration, blood was taken from the tail vein of each mouse and centrifuged to collect hematocyte fractions. After hemolysis of the hematocyte fractions, glycated hemoglobin (GHb) values were measured by affinity column chromatography with an automated glycated hemoglobin analyzer (TOSOH CORPORATION). Starting at the 27th day of repeated administration, mice were fasted for 16 hours, thereafter, blood was taken from the tail vein of each mouse and centrifuged to collect plasma. Plasma glucose levels were measured by the mutarotase-GOD method with a glucose assay kit (Glucose C2 Test Wako; Wako Pure Chemical Industries, Ltd.) Further, before and 27th day after the start of repeated administration, blood was taken from the tail vein of each mouse under non-fasting conditions and centrifuged to collect plasma. Plasma insulin levels were measured by ELISA with an insulin assay kit (Levis: Mouse Insulin ELISA KIT (H-Type); SHIBAYAGI Co., Ltd.)

### <Result 1>

The changes in GHb are expressed as the mean ± standard error in Table 1. The combination treatment of compound A (3 mg/kg) and metformin hydrochloride (300 mg/kg) markedly decreased the change in GHb, compared with each drug alone. A test by two-way ANOVA showed a significant interaction effect of compound A (3 mg/kg) and metformin hydrochloride (300 mg/kg) on the changes in GHb.

**Table 1**

| Group | Change in GHb (%) |
|---|---|
| Normal control | -0.05 ± 0.10 |
| Diabetic control | 1.20 ± 0.32 |
| Metformin hydrochloride, 100 mg/kg | 2.08 ± 0.33 |
| Metformin hydrochloride, 300 mg/kg | 1.70 ± 0.23 |
| Compound A, 3 mg/kg | 0.14 ± 0.39 |
| Compound A (3 mg/kg) + metformin hydrochloride (100 mg/kg) | 0.63 ± 0.27 |
| Compound A (3 mg/kg) + metformin hydrochloride (300 mg/kg) | -0.54 ± 0.15* |

| | |
|---|---|
| Change in GHb (%) = GHb (%) after repeated administration - GHb (%) before start of repeated administration *: P<0.05 for interaction effect of compound A (3 mg/kg) and metformin hydrochloride (300 mg/kg) (in two-way ANOVA) | |

### <Result 2>

Fasting plasma glucose levels are expressed as the mean ± standard error in Table 2. The Student's t-test was used to detect the difference between two groups. The combination treatment of compound A (3 mg/kg) and metformin hydrochloride (300 mg/kg) markedly decreased the fasting plasma glucose level, compared with each drug alone.

**Table 2**

| Group | Fasting plasma glucose level (mg/dL) |
|---|---|
| Normal control | 76.2 ± 2.2 |
| Diabetic control | 416.2 ± 24.4 |
| Metformin hydrochloride, 100 mg/kg | 378.3 ± 33.4 |
| Metformin hydrochloride, 300 mg/kg | 254.0 ± 24.7 |
| Compound A, 3 mg/kg | 275.0 ± 28.7 |
| Compound A (3 mg/kg) + metformin hydrochloride (100 mg/kg) | 226.8 ± 18.3 §§ |
| Compound A (3 mg/kg) + metformin hydrochloride (300 mg/kg) | 171.6±13.5 ††, # |

| | |
|---|---|
| §§: P<0.01 vs. metformin hydrochloride (100 mg/kg) group ††: P<0.01 vs. compound A (3 mg/kg) group #: P<0.05 vs. metformin hydrochloride (300 mg/kg) group. | |

### <Result 3>

The changes in non-fasting plasma insulin (IRI) levels are expressed as the mean ± standard error in Table 3. The Student's t-test was used to detect the difference between two groups. The combination treatment of compound A (3 mg/kg) and metformin hydrochloride (300 mg/kg) markedly inhibited the decrease in plasma IRI levels, compared with each drug alone.

**Table 3**

| Group | Change in plasma IRI level (ng/mL) |
|---|---|
| Normal control | -1.23 ± 0.70 |
| Diabetic control | -9.15 ± 1.05 |
| Metformin hydrochloride, 100 mg/kg | -9.81 ± 0.91 |
| Metformin hydrochloride, 300 mg/kg | -7.36 ± 1.29 |
| Compound A, 3 mg/kg | -5.01 ± 2.11 |
| Compound A (3 mg/kg) + metformin hydrochloride (100 mg/kg) | -5.07 ± 1.50 § |
| Compound A (3 mg/kg) + metformin hydrochloride (300 mg/kg) | 1.88 ± 1.87 †, ## |

| | |
|---|---|
| Change in plasma IRI level (ng/mL) = plasma IRI level (ng/mL) after repeated administration - plasma IRI level (ng/mL) before start of repeated administration §: P<0.05 vs. metformin hydrochloride (100 mg/kg) group t: P<0.05 vs. compound A (3 mg/kg) group ##: P<0.01 vs. metformin hydrochloride (300 mg/kg) group. | |

### Industrial Applicability

The present invention can provide superior pharmaceutical compositions for preventing or treating diabetes mellitus that exhibit an effective blood glucose lowering action in many diabetic patients and which yet cause fewer side effects. The present invention can also provide pharmaceutical compositions for preventing or treating various diabetic complications that result from hyperglycemia, such as diabetic retinopathy, diabetic nephropathy, diabetic neuropathy, cerebrovascular disorder, ischemic heart disease, and peripheral arterial disease.

## Claims

1. A pharmaceutical composition comprising a combination of
(A) (1S)-1,5-anhydro-1-[5-(4-ethoxybenzyl)-2-methoxy-4-methylphenyl]-1-thio-D-glucitol or a pharmaceutically acceptable salt thereof or a hydrate of the compound or the salt; and
(B) metformin.

2. The pharmaceutical composition according to claim 1, wherein the metformin is present in the form of metformin hydrochloride.

3. A combination of:
(A) (1S)-1,5-anhydro-1-[5-(4-ethoxybenzyl)-2-methoxy-4-methylphenyl]-1-thio-D-glucitol or a pharmaceutically acceptable salt thereof or a hydrate of the compound or the salt; and
(B) metformin
for use in a method of preventing or treating diabetes mellitus, diseases associated with diabetes mellitus, or complications of diabetes mellitus, wherein (A) and (B) are administered to a patient in need either simultaneously or separately.

4. The combination for use according to claim 3, wherein the metformin is present in the form of metformin hydrochloride.

5. The combination for use according to claim 3 or 4, wherein diabetes mellitus is type 2 diabetes.

6. The combination for use according to any one of claims 3 to 5, wherein the complication of diabetes mellitus is diabetic retinopathy, diabetic nephropathy, diabetic neuropathy, cerebrovascular disorder, ischemic cardiac disease, or peripheral arterial disease.

## Patentansprüche

1. Pharmazeutische Zusammensetzung, umfassend eine Kombination von
(A) (1S)-1,5-Anhydro-1-[5-(4-ethoxybenzyl)-2-methoxy-4-methylphenyl]-1-thio-D-glucitol oder ein pharmazeutisch annehmbares Salz davon oder ein Hydrat der Verbindung oder des Salzes; und
(B) Metformin.

2. Pharmazeutische Zusammensetzung gemäß Anspruch 1, worin das Metformin in Form von Metformin-Hydrochlorid vorliegt.

3. Kombination von:
(A) (1S)-1,5-Anhydro-1-[5-(4-ethoxybenzyl)-2-methoxy-4-methylphenyl]-1-thio-D-glucitol oder ein pharmazeutisch annehmbares Salz davon oder ein Hydrat der Verbindung oder des Salzes; und
(B) Metformin
zur Verwendung in einem Verfahren zum Verhindern oder Behandeln von Diabetes mellitus, von Erkrankungen, die mit Diabetes mellitus assoziiert sind, oder von Komplikationen von Diabetes mellitus, worin (A) und (B) einem bedürftigen Patienten entweder gleichzeitig oder separat verabreicht werden.

4. Kombination zur Verwendung gemäß Anspruch 3, worin das Metformin in Form von Metformin-Hydrochlorid vorliegt.

5. Kombination zur Verwendung gemäß Anspruch 3 oder 4, worin Diabetes mellitus Typ 2 Diabetes ist.

6. Kombination zur Verwendung gemäß irgendeinem der Ansprüche 3 bis 5, worin die Komplikation von Diabetes mellitus diabetische Retinopathie, diabetische Nephropathie, diabetische Neuropathie, zerebrovaskulare Störung, ischämische Herzerkrankung oder Peripher-Arterienerkrankung ist.

## Revendications

1. Composition pharmaceutique comprenant une combinaison de
(A) (1S)-1,5-anhydro-1-[5-(4-éthoxybenzyl)-2-méthoxy-4-méthylphényl]-1-thio-D-glucitol ou un sel pharmaceutiquement acceptable de celui-ci ou un hydrate du composé ou du sel ; et
(B) metformine.

2. Composition pharmaceutique selon la revendication 1, dans laquelle la metformine est présente sous la forme de chlorhydrate de metformine.

3. Combinaison de
(A) (1S)-1,5-anhydro-1-[5-(4-éthoxybenzyl)-2-méthoxy-4-méthylphényl]-1-thio-D-glucitol ou un sel pharmaceutiquement acceptable de celui-ci ou un hydrate du composé ou du sel ; et
(B) metformine
pour une utilisation dans une méthode de prévention ou de traitement du diabète sucré, de maladies associées au diabète sucré ou de complications du diabète sucré, dans laquelle (A) et (B) sont administrés à un patient en ayant besoin soit de manière simultanée, soit de manière séparée.

4. Combinaison pour une utilisation selon la revendication 3, dans laquelle la metformine est présente sous la forme de chlorhydrate de metformine.

5. Combinaison pour une utilisation selon la revendication 3 ou 4, dans laquelle le diabète sucré est un diabète de type 2.

6. Combinaison pour une utilisation selon l'une quelconque des revendications 3 à 5, dans laquelle la complication du diabète sucré est une rétinopathie diabétique, une néphropathie diabétique, une neuropathie diabétique, un trouble cérébrovasculaire, une maladie cardiaque ischémique ou une maladie artérielle périphérique.
